(19) Europäisches Patentamt
European Patent Office
Office européen des brevets

(11) Veröffentlichungsnummer: 0 368 008 A1

# (12) EUROPÄISCHE PATENTANMELDUNG

(21) Anmeldenummer: 89118838.5

(22) Anmeldetag: 11.10.89

(51) Int. Cl.5: C07C 43/23, C07C 65/21, C07C 205/37, C07C 235/60, C07C 323/20, C07C 323/62

(30) Priorität: 24.10.88 DE 3836175

(43) Veröffentlichungstag der Anmeldung: 16.05.90 Patentblatt 90/20

(84) Benannte Vertragsstaaten: DE FR GB IT

(71) Anmelder: BAYER AG
D-5090 Leverkusen 1 Bayerwerk(DE)

(72) Erfinder: Kysela, Ernst, Dr.
Virchowstrasse 14
D-5060 Bergisch Gladbach(DE)
Erfinder: Baasner, Bernd, Dr.
Hamberger Strasse 27d
D-5090 Leverkusen 3(DE)
Erfinder: Schaller, Klaus, Dr.
Am Sonnenschein 38
D-5600 Wuppertal 1(DE)

## (54) Fluor enthaltende Phenole.

(57) Fluor enthaltende Phenole der Formel

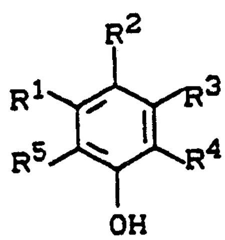

(I),

in der
einer der Reste $R^1$ und $R^2$ für $OCF_3$, $OCF_2CFClH$, $OCF_2CF_2H$, $SCF_2CF_2H$ oder $SCF_2CFClH$ und der andere der Reste $R^1$ und
$R^2$ für Wasserstoff oder Alkyl,
$R^3$ für Wasserstoff, Chlor oder $OCF_3$,
$R^4$ für Wasserstoff, Alkyl, $NO_2$, Chlor, $CONH_2$ oder COOH und
$R^5$ für Wasserstoff, Chlor oder $NO_2$ stehen,
wobei einer der Reste $R^1$ und $R^2$ auch für $SCF_3$ stehen kann, wenn $R^4$ für $NO_2$, $CONH_2$ oder COOH steht, und Verfahren zu deren Herstellung.

EP 0 368 008 A1

# Fluor enthaltende Phenole

Die vorliegende Erfindung betrifft neue, Fluor enthaltende Phenole der Formel (I)

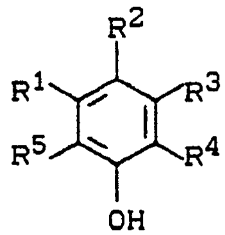

(I),

in der
einer der Reste $R^1$ und $R^2$ für $OCF_3$, $OCF_2CFClH$, $OCF_2CF_2H$, $SCF_2CF_2H$ oder $SCF_2CFClH$ und der andere der Reste $R^1$ und
$R^2$ für Wasserstoff oder $C_1$- bis $C_4$-Alkyl,
$R^3$ für Wasserstoff, Chlor oder $OCF_3$,
$R^4$ für Wasserstoff, $C_1$- bis $C_4$-Alkyl, $NO_2$, Chlor, $CONH_2$ oder COOH und
$R^5$ für Wasserstoff, Chlor oder $NO_2$ stehen,
wobei einer der Reste $R^1$ und $R^2$ auch für $SCF_3$ stehen kann, wenn $R^4$ für $NO_2$, $CONH_2$ oder COOH steht.

Soweit es sich bei den genannten Substituenten um $C_1$-bis $C_4$-Alkyl handelt ist Methyl und Ethyl bevorzugt, insbesondere Methyl.

Bevorzugt sind solche Verbindungen der Formel (I), bei denen
$R^1$ für Wasserstoff,
$R^2$ für $OCF_3$ oder $OCF_2CFClH$,
$R^3$ für Wasserstoff oder Chlor,
$R^4$ für Methyl, $NO_2$, Chlor oder $CONH_2$ und
$R^5$ für Wasserstoff, Chlor oder $NO_2$ stehen.

Beispiele für derartige bevorzugte Verbindungen sind solche, bei denen gilt (nicht genannte Reste = Wasserstoff):
$R^2 = OCF_3$, $R^4$ = Methyl,
$R^2 = OCF_2CFClH$, $R^4$ = Methyl,
$R^2 = OCF_3$, $R^4 = NO_2$,
$R^2 = OCF_3$, $R^3 = R^4 = R^5$ = Chlor,
$R^2 = OCF_3$, $R^4 = CONH_2$ und
$R^2 = OCF_3$, $R^4 = R^5 = NO_2$.

Besonders bevorzugt sind solche Verbindung der Formel (I), bei denen
$R^1$ für Wasserstoff,
$R^2$ für $OCF_3$ oder $OCF_2CFClH$,
$R^3$ für Wasserstoff oder Chlor,
$R^4$ für Methyl, $NO_2$ oder Chlor und
$R^5$ für Wasserstoff oder Chlor stehen.

Beispiele für derartige besonders bevorzugte Verbindungen sind solche, bei denen gilt (nicht genannte Reste = Wasserstoff):
$R^2 = OCF_3$, $R^4$ = Methyl,
$R^2 = OCF_2CFClH$, $R^4$ = Methyl,
$R^2 = OCF_3$, $R^4 = NO_2$ und
$R^2 = OCF_3$, $R^3 = R^4 = R^5$ = Chlor.

Eine ganz besonders bevorzugte Verbindung der Formel (I) ist 2-Methyl-4-trifluormethoxy-benzol.

Die erfindungsgemäßen, Fluor enthaltenden Phenole können auf verschiedene Weise hergestellt werden.

Beispielsweise kann man gemäß einer ersten Methode so vorgehen, daß man bei einem Phenol der Formel (II)

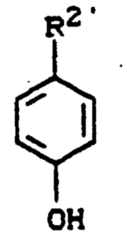

(II),

in der
$R^{2'}$ für $OCF_3$, $OCF_2CFClH$, $OCF_2CF_2H$, $SCF_2CFClH$ oder $SCF_2CF_2H$ steht
in die 2-Position und gegebenenfalls zusätzlich in die 3- und/oder 6-Position Substituenten einführt. Beim Einführen einer $NO_2$-, COOH- oder $CONH_2$-Gruppe kann im Ausgangsphenol der Formel (II) $R^{2'}$ auch für $SCF_3$ stehen.

Beispielsweise kann man so Chloratome durch Umsetzung mit einem Chlorierungsmittel einführen. Ein geeignetes Chlorierungsmittel ist z.B. elementares Chlor. Geeignete Temperaturen sind hierfür solche von beispielsweise 0 bis 50 °C. Man arbeitet zweckmäßigerweise in Gegenwart eines Katalysators, z.B. Eisen, und eines inerten Lösungsmittels, z.B. eines chlorierten Kohlenwasserstoffs.

Nitrogruppen kann man beispielsweise durch Umsetzung mit Salpetersäure einführen. Je nach Reaktionstemperatur und Konzentration der Salpetersäure lassen sich z.B. eine (in 2-Stellung) oder zwei Nitrogruppen (in 2- und 6-Stellung) einführen. Eine $NO_2$-Gruppe kann man beispielsweise mit 20 bis 33 gew.-%iger Salpetersäure bei 10 bis 30 °C, zwei $NO_2$-Gruppen beispielsweise mit 37 bis 60 gew.-%iger $HNO_3$ bei 50 bis 80 °C einführen.

Carboxylgruppen kann man beispielsweise durch Umsetzen mit Kohlendioxid unter erhöhtem Druck, bei erhöhter Temperatur und in Gegenwart einer Base einführen. Eine geeignete Base ist z.B. Kaliumcarbonat, geeignete Drucke beispielsweise solche von 10 bis 100 bar, geeignete Temperaturen beispielsweise solche von 150 bis 250 °C.

Säureamidgruppen kann man beispielsweise einführen, indem man gegebenenfalls wie oben beschriebenen eingeführte Carboxylgruppen beispielsweise zunächst in Gegenwart eines Lösungsmittels mit $SOCl_2$ und anschließend mit Ammoniak umsetzt.

Beispielsweise kann man gemäß einer zweiten Methode so vorgehen, daß man ein Anilin der Formel (III)

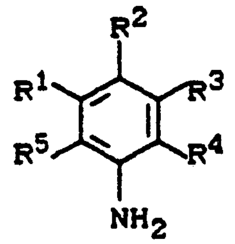

(III),

in der die Reste $R^1$ bis $R^5$ die bei Formel (I) angegebene Bedeutung haben, diazotiert und verkocht. Die Diazotierung kann man beispielsweise mit Natriumnitrit in Gegenwart von Salzsäure vornehmen, die Verkochung beispielsweise bei azeotroper Entfernung von Wasser in Gegenwart von Schwefelsäure bei Temperaturen um 100 bis 130 °C.

Beispielsweise kann man gemäß einer dritten Methode so vorgehen, daß man eine Verbindung der Formel (IV)

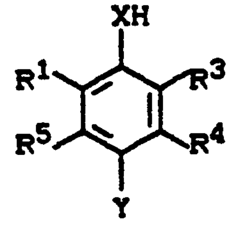

(IV),

in der
X für Sauerstoff oder Schwefel,
Y für OH, NHAcetyl oder $NO_2$ und
$R^1$ für Wasserstoff oder $C_1$- bis $C_4$-Alkyl stehen und
$R^3$ bis $R^5$ die bei Formel (I) angegebene Bedeutung haben
mit Tetrafluorethylen umsetzt und im Falle Y = NHAcetyl oder $NO_2$ diese Gruppen in an sich bekannter Weise in eine OH-Gruppe überführt. Falls X für Sauerstoff steht, steht Y vorzugsweise für NHAcetyl oder $NO_2$, falls X für Schwefel steht, steht Y vorzugsweise für OH.

Diese Methode eignet sich besonders zur Herstellung von Fluor enthaltenden Phenolen der Formel (I), bei denen $R^2$ für $OCF_2CF_2H$ oder $SCF_2CF_2H$ steht. Die Umsetzung mit Tetrafluorethylen erfolgt dabei vorzugsweise in Gegenwart einer Base und eines dipolaren, aprotischen Lösungsmittels beispielsweise bei Temperaturen im Bereich 50 bis 150 °C.

Es ist ausgesprochen überraschend, daß die erfindungsgemäßen Verbindungen der Formel (I) in so guten Ausbeuten und Selektivitäten zugänglich sind, denn bei den vielen denkbaren Substitutions-, Eliminierungs- und Additionsmöglichkeiten an den einzusetzenden Edukten und den erhaltenen Produkten war in großem Umfang mit dem Ablauf von unerwünschten Nebenreaktionen zu rechnen. Trotzdem können die erfindungsgemäßen Verbindungen der Formel (I) in Ausbeuten häufig von über 70 % der Theorie erhalten werden.

Die erfindungsgemäßen neuen Fluor enthaltenden Phenole der Formel (I) sind wichtige Zwischenprodukte. Man kann sie beispielsweise mit halogenierten Acetophenonen des Typs

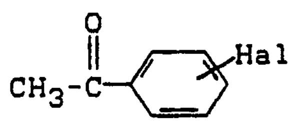

umsetzen zu Acetophenonen des Typs

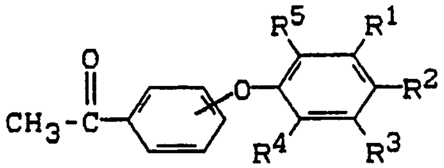

,

diese an der Methylgruppe halogenieren und durch Umsetzung der Halogenierungsprodukte mit Thioharnstoffderivaten des Typs

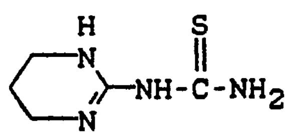

substituierte 2-Aminothiazole des Typs

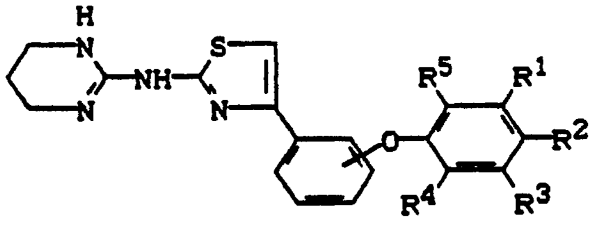

erhalten, die eine gute Wirksamkeit gegen Schädlinge, insbesondere gegen pflanzenschädigende Pilze besitzen.

Solche substituierten 2-Aminothiazole sind Gegenstand einer eigenen separaten Patentanmeldung.

Beispiele

Beispiel 1

3-[(α,α,ß,ß-Tetrafluorethyl)-thio]-phenol

126 g (1 Mol) 4-Mercaptophenol und 11,2 g (0,2 Mol) Kaliumhydroxid wurden in 300 ml Dimethylformamid gelöst. Bei 90 bis 100°C Innentemperatur wurde die Apparatur mit Stickstoff gespült und dann unter kräftigem Rühren Tetrafluorethylen eingeleitet. Sobald nichts mehr aufgenommen wurde wurden 75 Vol.-% des Dimethylformamids im Vakuum abgezogen, der Rückstand in 500 ml Eiswasser eingerührt, dieses auf einen pH-Wert von 3,5 gebracht, die organische Phase isoliert und destilliert. Es wurden 167 g Produkt mit einem Siedepunkt von 72 bis 73°C bei 17 mbar erhalten.

Beispiel 2

2,3,6-Trichlor-4-trifluormethoxy-phenol

89 g (0,5 Mol) 4-Trifluormethoxy-phenol wurden in 150 ml Tetrachlorkohlenstoff gelöst. Nach dem Zusatz von 0,5 g Eisenpulver wurde bei 40°C Chlor eingeleitet. Der Reaktionsverlauf wurde gaschromatographisch kontrolliert. Nach dem Erreichen eines 98 %igen Anteils an Trichlorprodukt wurde mit Stickstoff ausgeblasen und anschließend der Ansatz fraktioniert destilliert. Es wurden 106 g Produkt mit einem Siedepunkt von 120 bis 122°C bei 20 mbar erhalten, was einer Ausbeute von 74 % der Theorie entspricht. Der Schmelzpunkt des Produktes lag bei 56 bis 58°C.

Beispiel 3

2-Nitro-4-trifluormethoxy-phenol

53,4 g (0,3 Mol) 4-Trifluormethoxyphenol wurden bei 25°C im Verlaufe von 30 Minuten in 75 ml 30 %ige Salpetersäure eintropfen gelassen. Es wurde 2 Stunden bei 25°C nachgerührt, dann wurde der Ansatz in 500 ml Eiswasser gegossen und die organische Phase abgetrennt. Deren Destillation lieferte 56 g Produkt mit einem Kochpunkt von 102 bis 103°C bei 26 mbar, was einer Ausbeute von 83 % der Theorie entspricht.

Beispiel 4

2,6-Dinitro-4-trifluormethoxy-phenol

53,4 g (0,3 Mol) 4-Trifluormethoxyphenol wurden bei 60°C in 150 ml 40 %ige Salpetersäure im Verlaufe von 1 Stunde eintropfen gelassen. Es wurde noch 5 Stunden bei 60°C nachgerührt und dann wie in Beispiel 3 aufgearbeitet. Es wurden 63 g Produkt mit einem Siedepunkt von 126 bis 128°C bei 0,1 mbar erhalten, was 78 % der Theorie entspricht.

Beispiel 5

2-Hydroxy-5-trifluormethoxy-benzoesäure

178 g (1 Mol) 4-Trifluormethoxyphenol und 345 g (2,5 Mol) Kaliumcarbonat wurden in einem Autoklaven vorgelegt, danach 60 bar Kohlendioxid aufgepreßt und 4 Stunden auf 200 °C erwärmt. Nach dem Abkühlen und Entspannen wurde der Rückstand in 1,5 l heißem Wasser gelöst, heiß filtriert, das abgekühlte Filtrat mit Methylenchlorid extrahiert und mit Aktivkohle geklärt. Anschließend wurde ein pH von 1 eingestellt und das ausgefallene Produkt abgesaugt. Es wurden 192 g Produkt mit einem Schmelzpunkt von 129 °C erhalten, was 86 % der Theorie entspricht.

Beispiel 6

2-Hydroxy-5-trifluormethylthio-benzoesäure

Es wurde verfahren wie in Beispiel 5 beschrieben, jedoch wurden 194 g (1 Mol) 4-Trifluormethylmercapto-phenol eingesetzt und 185,7 g Produkt mit einem Schmelzpunkt von 122 bis 125 °C erhalten, was 78 % der Theorie entspricht.

Beispiel 7

2-Hydroxy-5-trifluormethoxy-benzamid

100 g (0,45 Mol) 2-Hydroxy-5-trifluormethoxy-benzoesäure (erhalten gemäß Beispiel 5) wurden in 350 ml Petrolether vorgelegt. Nach dem Zusatz von 1 ml Pyridin wurden im Verlaufe von 30 Minuten bei 30 °C 56 g $SOCl_2$ zutropfen gelassen und noch 5 Stunden bei 30 °C nachgerührt. Danach wurde von einem schmierigen Reaktionsrückstand abdekantiert und die abdekantierte Lösung mit Ammoniakgas gesättigt. Der ausfallende Feststoff wurde abfiltriert und getrocknet. Es wurden 82 g Produkt mit einem Schmelzpunkt von 188 bis 190 °C erhalten, was 75 % der Theorie entspricht.

Beispiel 8

3,4-Bis-trifluormethoxy-phenol

a) Herstellung des als Vorprodukt benötigten 3,4-Bistrifluormethoxy-anilins

415 g (3 Mol) Veratrol und 5 g Azobisisobutyronitril wurden in 3 l Tetrachlorkohlenstoff gelöst und auf Rückflußtemperatur erwärmt. Unter UV-Belichtung wurden 2,2 kg Chlor eingeleitet, was etwa 30 Stunden in Anspruch nahm, und gleichzeitig 20 g Azobisisobutyronitril gleichmäßig über den Reaktionszeitraum verteilt, zugegeben. Nach dem Reaktionsende wurde mit Stickstoff ausgeblasen, eingeengt und fraktioniert destilliert. Es wurden 650 g 92 %iges 1,2-Bistrichlormethoxy-benzol mit einem Siedepunkt von 106 bis 108 °C bei 0,2 mbar erhalten.

Zu 375 g so erhaltenem 1,2-Bistrichlormethoxy-benzol wurde bei -5 bis 0 °C 50 ml Fluorwasserstoff zutropfen gelassen, was 5 Stunden beanspruchte, 2,5 ml Antimonpentachlorid zugegeben und dann 12 Stunden lang auf 140 °C erwärmt. Der entstehende Chlorwasserstoff wurde bei 25 bar kontinuierlich entspannt. Nach dem Ende der Reaktion wurde überschüssiger Fluorwasserstoff abdestilliert, der Destillationsrückstand in 500 ml Wasser eingerührt und die organische Phase abgetrennt. Durch deren Fraktionierung wurden 178 g 98 %iges 1,2-Bistrifluormethoxy-benzol erhalten.

248 g so erhaltenes 1,2-Bistrifluormethoxybenzol wurden bei 0 °C in 250 g eines Gemisches aus 33 Gew.-% Salpetersäure und 67 Gew.-% Schwefelsäure im Verlaufe von 2 Stunden eintropfen gelassen. Danach wurde 5 Stunden bei 0 °C nachgerührt, anschließend auf Eiswasser gegeben, die organische Phase abgetrennt und destilliert. Es wurden 284 g 99 %iges 3,4-Bistrifluormethoxy-nitrobenzol erhalten.

291 g so erhaltenes 3,4-Bistrifluormethoxy-nitrobenzol wurden in 500 ml Methanol unter Zusatz von 10

g Raney-Nickel bei 40° C im Verlaufen von 3 Stunden mit 20 bar Wasserstoff hydriert. Nach dem Absaugen des Katalysators wurde fraktioniert destilliert und 242 g 3,4-Bistrifluormethoxy-anilin erhalten.

b) Herstellung von 3,4-Bistrifluormethoxy-phenol

356 g (1,36 Mol) 3,4-Bistrifluormethoxy-anilin wurden in 625 g Wasser und 332 g konzentrierter Salzsäure mit 246 g 40 %iger Natriumnitritlösung diazotiert. Die Diazoniumsalzlösung wurde in eine 120° C heiße Mischung aus 520 g Wasser, 968 g konzentrierter Schwefelsäure und 1200 g Xylol so zutropfen gelassen, daß dort durch azeotropes Abdestillieren des Wassers eine Innentemperatur von 120° C aufrechterhalten werden konnte. Anschließend wurde die Xylolphase abgetrennt und durch Extraktion mit Alkalilösung das entstandene 3,4-Bistrifluormethoxyphenol isoliert. Durch Destillation wurden 200 g Produkt mit einem Siedepunkt von 86 bis 88° C bei 25 mbar erhalten, was 53 % der Theorie entspricht.

Beispiel 9

3-(α,α,ß,ß-Tetrafluorethoxy)-4-methyl-phenol

a) Herstellung des als Vorprodukt benötigten 3-(α,α,ß,ß-Tetrafluorethoxy)-4-methyl-anilins

165 g 3-Hydroxy-4-methyl-acetanilid und 25 g Kaliumhydroxid wurden in 850 g Dimethylformamid auf 95° C erwärmt. Unter kräftigem Rühren wurden 120 g Tetrafluorethylen innerhalb von 5 Stunden eingeleitet. Nach wäßriger Aufarbeitung wurden 114 g 3-(α,α,ß,ß-Tetrafluorethoxy)-4-methyl-acetanilid erhalten. 265 g so hergestelltes 3-(α,α,ß,ß-Tetrafluorethoxy)-4-methyl-acetanilid wurden in 1,25 l Methanol mit 375 ml 25 %iger Natronlaugelösung versetzt und 12 Stunden auf 70° C erwärmt. Nachdem auf ca. 50 % des ursprünglichen Volumens eingeengt worden war wurde der Ansatz wäßrig aufgearbeitet. Die Destillation der organischen Phase lieferte 192 g 3-(α,α,ß,ß-Tetrafluorethoxy)-4-methoxyanilin.

b) Herstellung von 3-(α,α,ß,ß-Tetrafluorethoxy)-4-methyl-phenol

224 g 3-(α,α,ß,ß-Tetrafluorethoxy)-4-methyl-anilin wurden analog Beispiel 8 umgesetzt. Es wurden 153,5 g Produkt mit einem Siedepunkt von 78 bis 79° C bei 0,25 mbar erhalten, was 68 % der Theorie entspricht.

Beispiel 10

2-Methyl-4-trifluormethoxy-phenol

191 g (1 Mol) 2-Methyl-4-trifluormethoxy-anilin wurden analog Beispiel 8 umgesetzt. Es wurden 156 g Produkt mit einem Schmelzpunkt von 72° C und einem Siedepunkt von 80 bis 180° C bei 12 mbar erhalten, was 81 % der Theorie entspricht.

Beispiel 11

2-Methyl-4-(α,α,ß-Trifluor-ß-chlor-ethoxy)-phenol

238,5 g (1 Mol) 2-Methyl-4-(α,α,ß-Trifluor-ß-chlor-ethoxy)-anilin wurden analog Beispiel 8 umgesetzt. Es wurden 192,5 g Produkt mit einem Siedepunkt von 83 bis 84° C bei 0,35 mbar erhalten, was 80 % der Theorie entspricht.

## Ansprüche

1. Fluor enthaltende Phenole der Formel (I)

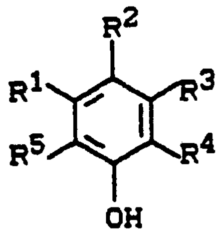

(I),

in der
einer der Reste $R^1$ und $R^2$ für $OCF_3$, $OCF_2CFClH$, $OCF_2CF_2H$, $SCF_2CF_2H$ oder $SCF_2CFClH$ und der andere der Reste $R^1$ und $R^2$ für Wasserstoff oder $C_1$- bis $C_4$-Alkyl,
$R^3$ für Wasserstoff, Chlor oder $OCF_3$,
$R^4$ für Wasserstoff, $C_1$- bis $C_4$-Alkyl, $NO_2$, Chlor, $CONH_2$ oder COOH und
$R^5$ für Wasserstoff, Chlor oder $NO_2$ stehen,
wobei einer der Reste $R^1$ und $R^2$ auch für $SCF_3$ stehen kann, wenn $R^4$ für $NO_2$, $CONH_2$ oder COOH steht.

2. Fluor enthaltende Phenole gemäß Anspruch 1, dadurch gezennzeiohnet, daß in Formel (I)
$R^1$ für Wasserstoff,
$R^2$ für $OCF_3$ oder $OCF_2CFClH$,
$R^3$ für Wasserstoff oder Chlor,
$R^4$ für Methyl, $NO_2$, Chlor oder $CONH_2$ und
$R^5$ für Wasserstoff, Chlor oder $NO_2$ stehen.

3. Fluor enthaltende Phenole gemäß Anspruch 1, dadurch gekennzeichnet, daß in Formel (I)
$R^1$ für Wasserstoff,
$R^2$ für $OCF_3$ oder $OCF_2CFClH$,
$R^3$ für Wasserstoff oder Chlor,
$R^4$ für Methyl, $NO_2$ oder Chlor und
$R^5$ für Wasserstoff oder Chlor stehen.

4. Verfahren zur Herstellung von Fluor enthaltenden Phenolen der Formmel (I)

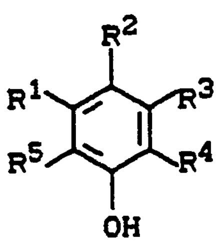

(I),

in der
einer der Reste $R^1$ und $R^2$ für $OCF_3$, $OCF_2CFClH$, $OCF_2CF_2H$, $SCF_2CF_2H$ oder $SCF_2CFClH$ und der andere der Reste $R^1$ und
$R^2$ für Wasserstoff oder $C_1$- bis $C_4$-Alkyl,
$R^3$ für Wasserstoff, Chlor oder $OCF_3$,
$R^4$ für Wasserstoff, $C_1$- bis $C_4$-Alkyl, $NO_2$, Chlor, $CONH_2$ oder COOH und
$R^5$ für Wasserstoff, Chlor oder $NO_2$ stehen,
wobei einer der Reste $R^1$ und $R^2$ auch für $SCF_3$ stehen kann, wenn $R^4$ für $NO_2$, $CONH_2$ oder COOH steht,
dadurch gekennzeichnet, daß man bei einem Phenol der Formel (II)

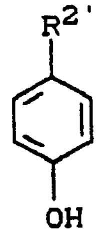

(II),

in der
$R^2$ für $OCF_3$, $OCF_2CFClH$, $OCF_2CF_2H$, $SCF_2CFClH$ oder $SCF_2CF_2H$ steht
in die 2-Position und gegebenenfalls zusätzlich in die 3- und/oder 6-Position Substituenten einführt, wobei beim Einführen einer $NO_2$-, COOH- oder $CONH_2$-Gruppe kann im Ausgangsphenol der Formel (II) $R^2$ auch für $SCF_3$ stehen kann.

5. Verfahren zur Herstellung von Fluor enthaltenden Phenolen der Formel (I)

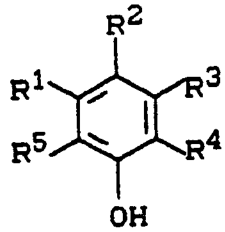

(I),

in der
einer der Reste $R^1$ und $R^2$ für $OCF_3$, $OCF_2CFClH$, $OCF_2CF_2H$, $SCF_2CF_2H$ oder $SCF_2CFClH$ und der andere der Reste $R^1$ und $R^2$ für Wasserstoff oder $C_1$- bis $C_4$-Alkyl,
$R^3$ für Wasserstoff, Chlor oder $OCF_3$,
$R^4$ für Wasserstoff, $C_1$- bis $C_4$-Alkyl, $NO_2$, Chlor, $CONH_2$ oder COOH und
$R^5$ für Wasserstoff, Chlor oder $NO_2$ stehen,
wobei einer der Reste $R^1$ und $R^2$ auch für $SCF_3$ stehen kann, wenn $R^4$ für $NO_2$, $CONH_2$ oder COOH steht,
dadurch gekennzeichnet, daß man ein Anilin der Formel (III)

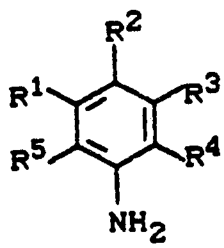

(III),

in der die Reste $R^1$ bis $R^5$ die bei Formel (I) angegebene Bedeutung haben, diazotiert und verkocht.

6. Verfahren zur Herstellung von Fluor enthaltenden Phenolen der Formel (I)

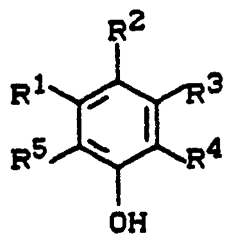

(I),

in der

einer der Reste $R^1$ und $R^2$ für $OCF_3$, $OCF_2CFClH$, $OCF_2CF_2H$, $SCF_2CF_2H$ oder $SCF_2CFClH$ und der andere der Reste $R^1$ und
$R^2$ für Wasserstoff oder $C_1$- bis $C_4$-Alkyl,
$R^3$ für Wasserstoff, Chlor oder $OCF_3$,
$R^4$ für Wasserstoff, $C_1$- bis $C_4$-Alkyl, $NO_2$, Chlor, $CONH_2$ oder COOH und
$R^5$ für Wasserstoff, Chlor oder $NO_2$ stehen,
wobei einer der Reste $R^1$ und $R^2$ auch für $SCF_3$ stehen kann, wenn $R^4$ für $NO_2$, $CONH_2$ oder COOH steht, dadurch gekennzeichnet, daß man eine Verbindung der Formel (IV)

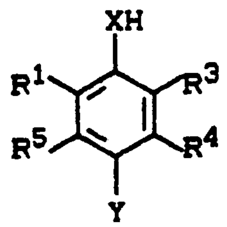

(IV),

in der
X für Sauerstoff oder Schwefel,
Y für OH, NHAcetyl oder $NO_2$ und
$R^1$ für Wasserstoff oder $C_1$- bis $C_4$-Alkyl stehen und
$R^3$ bis $R^5$ die bei Formel (I) angegebene Bedeutung haben
mit Tetrafluorethylen umsetzt und im Falle Y = NHAcetyl oder $NO_2$ diese Gruppen in an sich bekannter Weise in eine OH-Gruppe überführt.

Europäisches Patentamt

# EUROPÄISCHER RECHERCHENBERICHT

Nummer der Anmeldung

EP 89 11 8838

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 091 598 (BASF)<br>* Seiten 7-10 *<br>--- | 1-6 | C 07 C 43/23<br>C 07 C 65/21<br>C 07 C 205/37<br>C 07 C 235/60<br>C 07 C 323/20<br>C 07 C 323/62 |
| X | EP-A-0 054 149 (BASF)<br>* Seiten 6-17 *<br>--- | 1-4 | |
| X | EP-A-0 175 188 (NIHON TOKUSHU NOYAKO SEIZO)<br>* Seiten 31,32 *<br>--- | 1-4 | |
| X | DE-A-2 312 906 (FARBWERKE HOECHST)<br>* Patentansprüche; Seiten 6-8 *<br>--- | 1-3 | |
| X | DE-A-3 709 609 (HOECHST)<br>* Beispiele 5-8; Patentansprüche *<br>----- | 1-3,5 | |
| | | | **RECHERCHIERTE SACHGEBIETE (Int. Cl.5)** |
| | | | C 07 C 43/00<br>C 07 C 65/00<br>C 07 C 205/37<br>C 07 C 235/00<br>C 07 C 323/00 |

Der vorliegende Recherchenbericht wurde für alle Patentansprüche erstellt

| Recherchenort | Abschlußdatum der Recherche | Prüfer |
|---|---|---|
| DEN HAAG | 02-02-1990 | WRIGHT M.W. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)